# FASCICULE DE BREVET EUROPEEN

(11) **EP 2 941 234 B1**
(45) Date de publication et mention de la délivrance du brevet: **04.04.2018**
(21) Numéro de dépôt: 14700038.4
(22) Date de dépôt: 02.01.2014
(51) Int. Cl.: A61G 7/057, A61B 5/11

(54) **DISPOSITIF DE SUPPORT POUR SUPPORTER UN CORPS, EN PARTICULIER UN CORPS HUMAIN**
TRÄGERVORRICHTUNG ZUM UNTERSTÜTZEN EINES KÖRPERS, INSBESONDERE EINES MENSCHLICHEN KÖRPERS
SUPPORT DEVICE FOR SUPPORTING A BODY, IN PARTICULAR A HUMAN BODY

(30) Priorité: 04.01.2013 FR 1350044
(43) Date de publication de la demande: 11.11.2015
(73) Titulaire: System Assistance Medical, 47300 Ledat (FR)
(72) Inventeur: CINQUIN, Sébastien, F-47150 La Sauvetat sur Lede (FR)
(74) Mandataire: Casalonga
(86) Numéro de dépôt international: PCT/EP2014/050002
(87) Numéro de publication internationale: WO 2014/106624

(56) Documents cités:
- WO-A1-93/09749
- WO-A1-2008/138074
- US-A- 6 009 580
- US-A1- 2011 302 719
- US-B1- 7 378 975

## Description

La présente invention concerne le domaine des dispositifs de support pour supporter un corps ou une partie d'un corps, en particulier d'un corps humain, destinés à former au moins en partie des coussins ou des matelas et à être utilisés en particulier dans le domaine médical pour le confort des patients en position allongée ou assise, notamment pour la prévention des escarres.

Le brevet EP 0 676 158 décrit un matelas pneumatique comprenant une pluralité de boudins verticaux gonflables et équipé de moyens de détection de déformations qui comprend une feuille métallique placée sur la face supérieure du matelas et une feuille munie d'un élément inductif tel qu'une bobine d'induction placée sous le matelas, la feuille métallique et la bobine d'induction recouvrant une zone incluant plusieurs boudins gonflables, ainsi qu'un circuit électronique relié à l'élément inductif pour délivrer un signal électrique correspondant à la distance entre la feuille métallique et la bobine d'induction, qui varie selon l'écrasement du matelas. Un asservissement d'un moyen de gonflage du matelas audit signal électrique peut permettre de réguler à une valeur de consigne la distance entre la feuille métallique et la bobine d'induction.

Le brevet WO 98/27850 décrit un ensemble comprenant un matelas et, au-dessous le matelas, un moyen de détection de déformations qui s'étend sur une zone du matelas et qui comprend un élément d'espacement d'épaisseur variable, une feuille métallique entre le matelas et cet élément d'espacement et un élément variateur d'impédance sur la face inférieure de cet élément d'espacement. Les déformations du matelas sous l'effet de la charge induisent des déformations de l'élément d'espacement qui sont mesurées par les variations de l'impédance de l'élément variateur d'impédance.

Le brevet WO 99/04673 décrit un coussin formé d'une pluralité de cellules gonflables en saillie vers le haut par rapport à une feuille d'embase et équipé de capteurs individuels de déformations qui sont logés respectivement à l'intérieur des cellules. Chaque capteur comprend un élément élastiquement déformable et un interrupteur soumis à cet élément déformable ou bien un capteur photoélectrique à réflexion.

Le document US 6 009 580 décrit un matelas composé de boudins adjacents s'étendant sur toute la largeur du matelas. Ce matelas est équipé d'un détecteur d'enfoncement qui comprend, sur une zone centrale couvrant plusieurs boudins adjacents, un élément variateur d'impédance tel qu'une self-induction située sous le matelas et une feuille métallique située sur le matelas.

Les dispositions connues décrites ci-dessus ne donnent pas entière satisfaction. En particulier, la détection de l'enfoncement du matelas est imprécise et peu fiable. La mise en place, le maintien et le remplacement des moyens de détection sont difficiles et onéreux. En outre, les dispositifs connus ne sont pas applicables pour détecter l'enfoncement de supports quelconques.

Le but de la présente invention est de proposer un perfectionnement aux dispositifs de support.

Il est proposé un dispositif de support pour supporter un corps ou une partie d'un corps, en particulier d'un corps humain, comprenant au moins un élément déformable présentant une première face et une seconde face opposées et équipé d'au moins un moyen de détection de déformations dudit élément déformable.

Selon un mode de réalisation, l'élément déformable comprend une feuille d'embase et au moins une pluralité de cellules creuses gonflables en saillie d'un côté de la feuille d'embase et individuellement déformables en hauteur par rapport à la feuille d'embase ; le moyen de détection comprend une feuille de substrat associée à ladite feuille d'embase, s'étendant parallèlement à celle-ci, et munie d'une pluralité d'enroulements métalliques formés à plat sur au moins une face de cette feuille de substrat, et une pluralité de pastilles métalliques solidaires respectivement des parties d'extrémité desdites cellules creuses gonflables, éloignées de ladite feuille d'embase ; les pastilles métalliques sont respectivement situées au-dessus des parties centrales des enroulements métalliques.

Le moyen de détection de déformations peut comprendre un moyen de détection inductive par impulsion relié auxdits enroulements.

Le moyen de détection de déformations peut comprendre un moyen permettant de relier séquentiellement et successivement les enroulements ou des groupes d'enroulements audit moyen de détection inductive par impulsion.

Les enroulements peuvent être divisés en groupes d'enroulements dans lesquels les enroulements sont électriquement branchés en série.

Les pastilles métalliques peuvent être portées par au moins une feuille souple s'étendant au-dessus des parties d'extrémité desdites cellules creuses gonflables.

Les pastilles métalliques peuvent être fixées sur les parties d'extrémité desdites cellules creuses gonflables.

Les pastilles métalliques peuvent être fixées sur une face intérieure des cellules creuses, parallèle à ladite feuille d'embase.

La feuille de substrat peut être située sur le côté de la feuille d'embase opposé auxdites cellules creuses.

Les espaces intérieurs peuvent être reliés par l'intermédiaire de canaux aménagés dans la feuille d'embase.

L'élément de gonflage peut comprendre une première et une seconde feuilles accolées formant ladite feuille d'embase et une pluralité de protubérances creuses reliées à ladite seconde feuille et délimitant lesdites cellules creuses, au moins l'une des feuilles étant conformée de façon à former des canaux de communication entre les espaces intérieurs des cellules creuses.

L'élément déformable peut comprendre un bloc élastiquement déformable, les pastilles métalliques étant portées par au moins une feuille souple.

Il est également proposé un ensemble comprenant le dispositif de support gonflable, et comprenant un système de pompe et d'échappement relié à un moyen de branchement extérieur relié auxdites cellules creuses par des canaux inclus dans la feuille d'embase et une unité électronique de détection inductive par impulsion reliée à des moyens de connexion électrique extérieure reliés auxdits enroulements et de gestion de l'activation du système de pompe et d'échappement.

Il est également proposé un ensemble comprenant un dispositif de support et comprenant une unité électronique de détection inductive par impulsion reliée à des moyens de connexion électrique extérieure reliés auxdits enroulements et de gestion d'une alarme.

Des dispositifs de support et ses accessoires vont maintenant être décrits à titre d'exemples non limitatifs, illustrés par le dessin sur lequel :
- la figure 1 représente, en perspective, un matelas de literie équipé d'un dispositif de support muni d'un moyen de détection de déformations ;
- la figure 2 représente une coupe verticale partielle schématique du matelas de literie équipé d'un dispositif de support de la figure 1 et de moyens de gestion de ce dernier ;
- la figure 3 représente une vue en perspective éclatée du dispositif de support et d'une feuille de substrat associée ;
- la figure 4 représente une vue de dessus du dispositif de support ;
- la figure 5 représente une coupe verticale partielle détaillée du matelas de literie équipé du dispositif de support des figures 3 et 4 ;
- la figure 6 représente une vue détaillée d'une face de la feuille de substrat de la figure 3 ;
- la figure 7 représente un schéma électronique d'une unité de gestion du dispositif de support ;
- la figure 8 représente une variante de réalisation, selon une coupe correspondant à celle de la figure 5 ; et
- la figure 9 représente un autre dispositif de support équipé d'un autre moyen de détection de déformations.

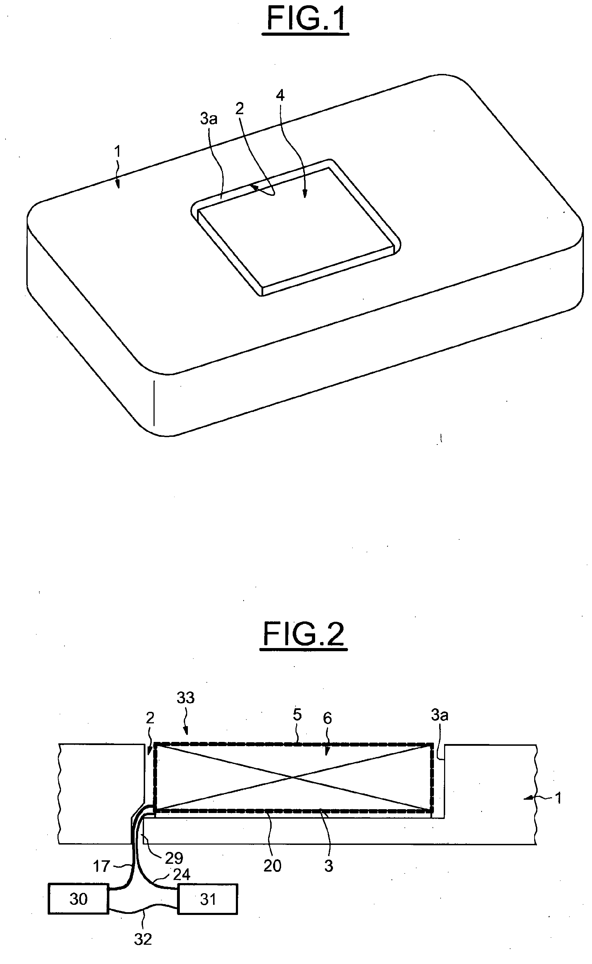

Comme illustré sur les figures 1 et 2, un matelas 1 de literie présente un évidement 2 ouvert vers le haut et qui présente un fond 3 par exemple rectangulaire ou carré et une paroi périphérique 3a. Par exemple, l'évidement 2 est réalisé dans une partie centrale du matelas 1, dans une zone allant du milieu de la cuisse au milieu du dos d'un utilisateur allongé sur le matelas 1.

A l'intérieur de cet évidement 2 est installé un dispositif de support 4 qui occupe sensiblement tout le volume de l'évidement 2 et dont la face supérieure est sensiblement dans le plan de la face supérieure du matelas 1.

Le dispositif de support 4 comprend, par exemple enveloppé dans une housse souple 5, un élément déformable gonflable 6 de forme générale parallélépipédique.

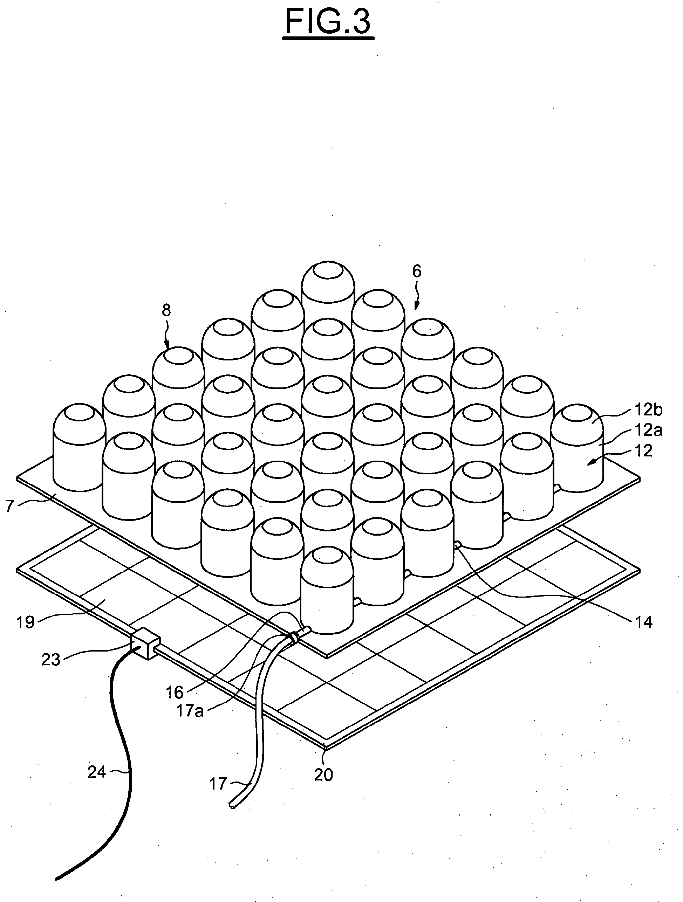

Comme illustré sur les figures 3 à 5, l'élément gonflable 6, par exemple en une matière plastique ou caoutchouteuse souple, comprend une feuille d'embase 7 adjacente et parallèle au fond 3 de l'évidement 2 du matelas 1 et une pluralité de cellules creuses gonflables 8 en saillie vers le haut d'un côté de la feuille d'embase 7, réparties dans le sens de la largeur et dans le sens de la longueur du matelas et individuellement déformables en particulier en hauteur par rapport à la feuille d'embase 7.

Par exemple, la feuille d'embase 7 recouvre sensiblement tout le fond 3 de l'évidement 2 du matelas 1 et est sensiblement rectangulaire. Les cellules creuses 8, substantiellement cylindriques, sont par exemple réparties sensiblement sur toute la surface de la feuille d'embase 7 selon une matrice carrée et sont situées à faible distance les unes des autres. Selon une variante de réalisation, les cellules creuses 8 pourraient être réparties selon une matrice en losange. Selon l'exemple représenté, sont prévues trente six cellules disposées sur six colonnes et six lignes orthogonales.

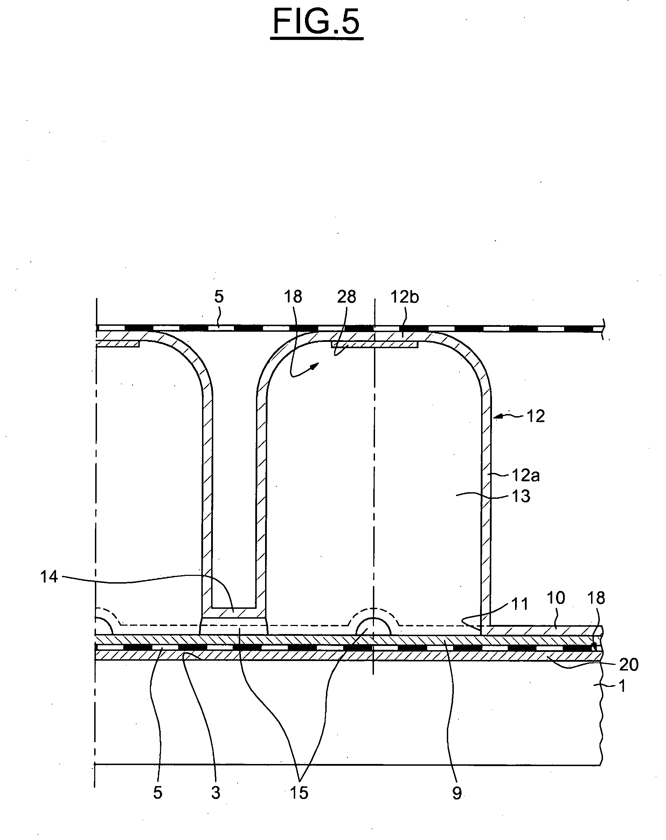

Comme illustré plus particulièrement sur la figure 5, l'élément gonflable 6 est constitué d'une première partie formée par une première feuille 9 et d'une seconde partie formée, d'une seule pièce, par une seconde feuille 10 présentant une pluralité d'ouvertures 11 et par une pluralité de protubérances creuses 12 en saillie vers le haut depuis les bords des ouvertures 11. La première feuille 9 et la seconde feuille 10 sont accolées et fixées entre elles par collage et forment la feuille d'embase 7. Les protubérances creuses 12 et les parties correspondantes de la première feuille 9 délimitent les espaces intérieurs 13 des cellules creuses 8.

Les protubérances creuses 12 présentent une paroi périphérique 12a de section circulaire ou en étoile et une paroi d'extrémité supérieure 12b présentant une partie centrale plate.

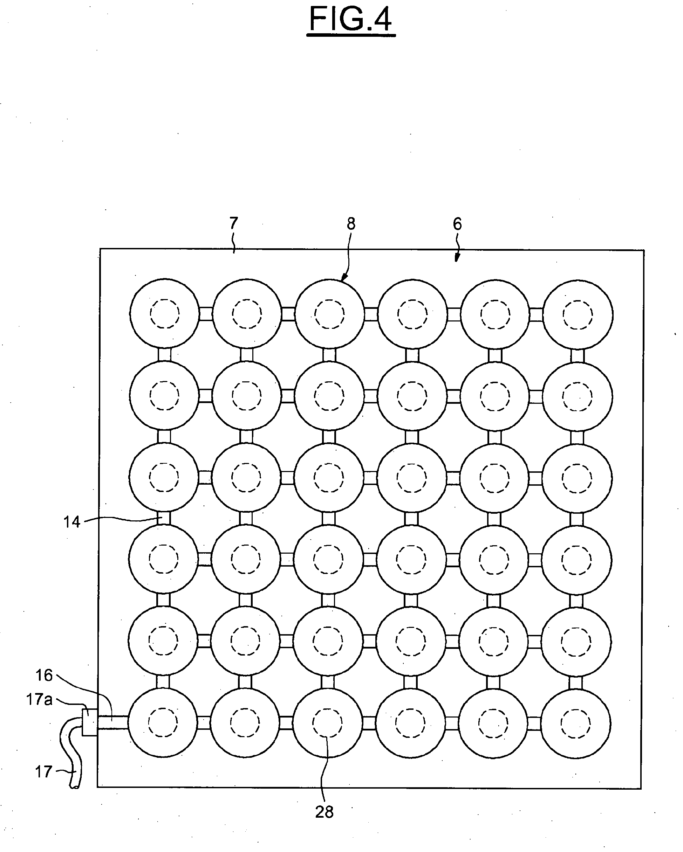

La seconde feuille 10 présente une pluralité de faibles déformations 14 vers le haut qui, avec les parties correspondantes de la première feuille 9, délimitent des canaux de communication 15 reliant les espaces intérieurs 13 des cellules creuses 8 adjacentes. En outre (figures 3 et 4), la seconde feuille 10 présente une faible déformation 16 vers le haut qui, avec une partie correspondante de la première feuille 9, délimite un canal de communication dont l'une des extrémités débouche dans une cellule creuse et dont l'autre extrémité aménagée sur un bord de la feuille d'embase 7 peut être reliée à un tuyau de branchement 17 par un moyen de branchement étanche 17a.

Le dispositif de support 4 est équipé d'un moyen de détection 18 des déformations des cellules creuses 8, dans le sens de leur hauteur.

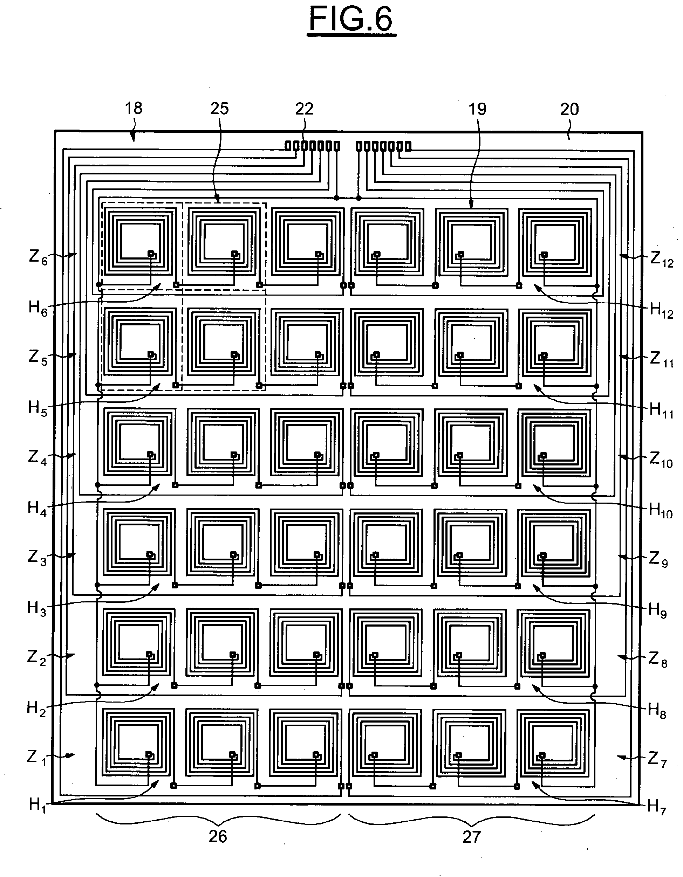

Comme illustré sur les figures 3 et 6, le moyen de détection 18 comprend une pluralité d'enroulements métalliques 19 sérigraphiés à plat sur des zones d'une face d'une feuille de substrat 20, de préférence en une matière plastique souple.

Comme illustré en particulier sur les figures 2 et 5, la feuille de substrat 20 est associée à une face de l'élément gonflable 6 et est, plus précisément, interposée entre la feuille d'embase 7 de l'élément gonflable 6 et le fond 3 de l'évidement 2 du matelas 1 et parallèle à cette feuille d'embase 7. Par exemple, la périphérie de la feuille de substrat 20 coïncide avec celle de la feuille d'embase 7.

Comme illustré sur les figures 3 et 6, les enroulements métalliques 19 sont reliés sélectivement, par des pistes de connexion électrique 21, à des plots de connexion électrique extérieure 22 aménagés à proximité les uns des autres et à proximité d'un bord de la feuille de substrat 20, les pistes de connexion électrique 21 et les plots de connexion électrique 22 étant sérigraphiés sur au moins une face de la feuille de substrat 20.

Sur les plots de connexion électrique 22 peut être accouplé un connecteur 23 d'extrémité d'un câble de connexion électrique 24.

Selon l'exemple particulier de réalisation illustré sur la figure 6, les enroulements métalliques 19 sont formés sur des zones adjacentes 25 de la feuille de substrat 20 et sont répartis selon une matrice carrée. Selon une variante de réalisation, les enroulements métalliques 19 pourraient être répartis selon une matrice en losange. Selon l'exemple représenté, sont prévues trente six zones 25 disposées sur six colonnes et six lignes orthogonales.

La surface totale couverte par les cellules creuses 8 peut coïncider sensiblement avec la surface totale couverte par les enroulements métalliques 19, sans que cela soit forcément obligatoire. Le nombre de cellules creuses 8 peut être égal au nombre d'enroulements métalliques 19, sans que cela soit forcément obligatoire.

Sur chaque ligne de ladite matrice de zones adjacentes 25, les six enroulements métalliques 19 sont divisés en deux groupes 26 et 27 de trois enroulements métalliques qui sont reliés en séries et reliés à des plots de connexion électrique 22 correspondants. De plus, dans chacun de ces groupes, les enroulements métalliques 19 sont enroulés dans le même sens et, d'un groupe à l'autre, les enroulements métalliques 19 sont enroulés en sens inverse. En outre, les six groupes 26 et les six groupes 27, adjacents dans le sens des colonnes de ladite matrice, sont enroulés dans le même sens. Ainsi, les groupes d'enroulements 26 et 27 déterminent douze zones de détection Z₁ à Z₁₂ réparties sur la surface de la feuille de substrat 20, dont six zones de détection Z₁ à Z₆ sur une colonne et six zones de détection Z₇ à Z₁₂, munies respectivement d'éléments inductifs H₁ à H₁₂ composés chacun de trois enroulements 19 branchés en série.

Comme illustré sur la figure 5, le moyen de détection 18 comprend en outre une pluralité de pastilles métalliques 28, en une ou plusieurs parties, qui sont associées à une autre face de l'élément gonflable et qui sont, plus précisément, fixées sur les parties d'extrémité des cellules creuses 8, éloignées de ladite feuille d'embase 7 et à distance des enroulements métalliques 19 portés par la feuille de substrat 20. Par exemple, les pastilles métalliques 28 sont à l'intérieur des cellules creuses 8 et sont respectivement collées sur la face intérieure des parties centrales plates des parois d'extrémité supérieures 12b des protubérances creuses 12, de telle sorte qu'elles sont protégées contre toute agression extérieure. Dans ce cas, les pastilles métalliques 28 sont mises en place avant collage des feuilles 9 et 10 formant la feuille d'embase 7.

Dans le cas de la variante de réalisation décrite précédemment, les pastilles métalliques 28 peuvent être placées au-dessus des parties centrales des enroulements 19, bien que cela ne soit pas obligatoire.

Comme illustré sur la figure 2, le matelas 1 présente un passage traversant 29 débouchant dans l'évidement 2 et ouvert au-dessous du matelas. Le tuyau de branchement 17 et le câble de connexion électrique 24 s'étendent au travers du passage 29.

Le tuyau de branchement 17 est relié à un système de pompe et d'échappement 30 et le câble de connexion électrique 24 est relié à une unité électronique 31, le système de pompe et d'échappement 30 et le câble de connexion électrique 24 étant reliés par un câble électrique 32. Ainsi, le dispositif de support 4, la feuille de substrat 20 munie des enroulements métalliques 19, le système de pompe et d'échappement 30 et l'unité électronique 31 constituent un ensemble fonctionnel 33.

Cet ensemble fonctionnel 33 peut fonctionner de la manière suivante.

L'élément gonflable 6 est en appui au-dessus de la feuille de substrat 3 grâce à sa feuille d'embase 7 et la feuille de substrat 3 est en appui au-dessus du fond 3 de l'évidement 2 du matelas 1.

Lorsque le corps d'un utilisateur exerce une charge sur les parties supérieures d'extrémité d'au moins une partie des cellules creuses 8, les cellules creuses 8 qui sont sollicitées sont enfoncées respectivement en fonction des pressions locales de soutien résultant de la pression interne produite dans les espaces intérieurs 13 des cellules creuses 8 par le système de pompe et d'échappement 30.

Les pastilles métalliques 28 suivant les mouvements des parties d'extrémité des cellules creuses 8 desquelles elles sont solidaires, la mesure ou la détermination de la valeur d'un ou de plusieurs signaux électriques, représentatifs du couplage électromagnétique entre les pastilles métalliques 28 et les enroulements métalliques 19 alimentés en énergie électrique, d'une manière adaptée, par l'unité électronique 31, constituent un moyen témoignant des effets de la charge sur les cellules creuses 8 ou du degré de déformation de ces dernières.

En faisant varier la pression à l'intérieur des cellules creuses 8, grâce à des réglages ou des régulations du système de pompe et d'échappement 30, il est possible de faire varier le taux de déformation ou d'enfoncement des cellules creuses 8 et les pressions locales des cellules creuses 8 exercées sur le corps de l'utilisateur.

Selon un exemple particulier de réalisation, l'unité électronique 31 est programmée de façon à réaliser une détection inductive par impulsion de la position des pastilles métalliques 25 par rapport aux enroulements métalliques 19, et plus particulièrement par rapport respectivement aux enroulements 19 inclus dans les zones de détection Z₁ à Z₁₂.

Comme il est connu dans le domaine de l'électronique, une détection inductive par impulsion se produit de la manière suivante. Un enroulement constituant un élément inductif ou une self est parcouru par un courant important pendant un bref instant correspondant à la durée d'une impulsion de tension d'alimentation. A la suite de cette impulsion de tension, il se crée par induction une surtension aux bornes de l'enroulement, cette surtension diminuant très rapidement jusqu'à la valeur nominale de la tension d'alimentation. Lorsqu'un objet métallique est approché de l'enroulement, la durée du retour à cette valeur nominale est augmentée, d'autant plus que l'objet est plus proche de l'enroulement. En conséquence, mesurer ou déterminer cette durée de retour ou la durée de l'impulsion résultante de tension incluant l'impulsion de tension d'alimentation et la durée de retour permet de mesurer ou déterminer la distance entre l'enroulement et l'objet.

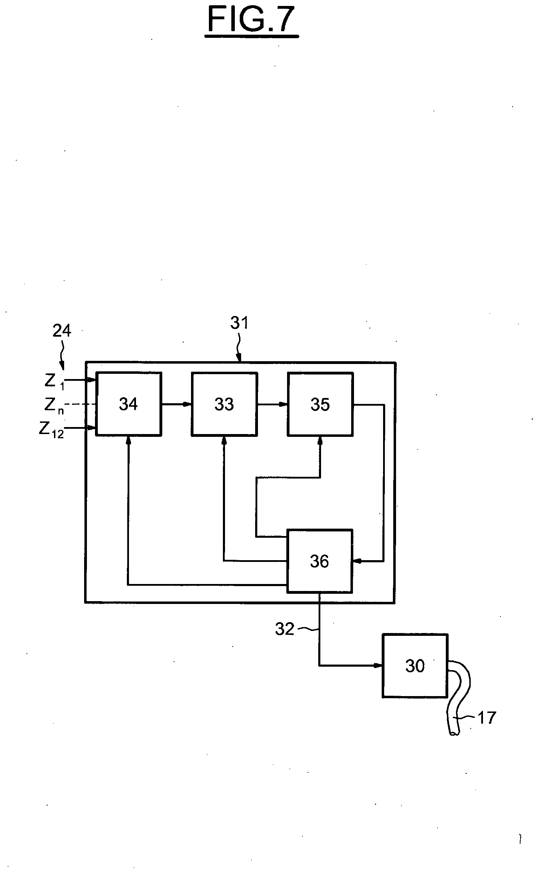

Comme illustré schématiquement sur la figure 7, l'unité électronique 31, pour appliquer ce qui précède, peut comprendre notamment :
- un circuit 33 de mesure de ladite durée de retour ou de ladite durée de l'impulsion résultante de tension,
- un circuit de démultiplexage 34 pour relier sélectivement chacun des éléments inductifs au circuit de mesure,
- un circuit de comparaison 35 de ladite durée de retour ou de la durée de l'impulsion résultante de tension à un seuil REF1, par exemple réglable, dont la valeur correspond à une durée prédéterminée de retour ou une durée prédéterminée de ladite impulsion résultante de tension,
- et un circuit logique programmé 36, ou microprocesseur, pour commander, selon des cycles de mesure, lesdits circuits 33, 34 et 35 de façon à obtenir successivement les résultats des comparaisons correspondants aux éléments inductifs H₁ à H₁₂ et gérer le fonctionnement du système de pompe et d'échappement 30 en fonction des résultats des comparaisons.

Considérant l'exemple de la disposition précitée des pastilles métalliques 28 par rapport aux enroulements métalliques 19, on peut estimer approximativement que chaque pastille métallique 28 influence l'enroulement 19 situé au-dessous.

Pour chaque élément inductif H₁ à H₁₂, la durée de retour ou la durée de ladite impulsion résultante de tension fournissent une information en ce qui concerne la distance entre les pastilles métalliques 28 situées au-dessus de l'élément inductif considéré. Cette distance prédéterminée intègre en fait, pour chaque élément inductif, des distances relatives de l'une, deux ou des trois pastilles métalliques 28 par rapport aux enroulements métalliques 19 le composant.

La comparaison de la durée de retour ou la durée de ladite impulsion résultante de tension avec le seuil REF1 fournit une information en ce qui concerne la position des pastilles métalliques 28 situées au-dessus de l'élément inductif considéré par rapport à la feuille de substrat 20. Cette comparaison permet de déterminer si les enfoncements ou les déformations de l'une, deux ou les trois cellules creuses 8 sont au-delà ou en deçà d'une distance prédéterminée associée au seuil REF1, par rapport à l'élément inductif considéré composé des enroulements 19 correspondants.

Pour tenir compte de ladite comparaison, le circuit logique 36 peut être programmé pour commander le système de pompe et d'échappement 30 de façon à regonfler l'élément gonflable 8 si le seuil REF est franchit dans le sens du dégonflage de ce dernier.

Un second seuil REF2 peut être également réglé et le circuit logique 36 peut être programmé pour commander le système de pompe et d'échappement 30 de façon à dégonfler l'élément gonflable 8 si ce second seuil REF2 est franchit dans le sens du gonflage de ce dernier.

Le circuit logique 36 peut être programmé pour commander le système de pompe et d'échappement 30 de façon à maintenir l'état du gonflage de l'élément gonflable 8 de façon à maintenir la distance précitée en deux valeurs extrêmes correspondant au premier seuil REF1 et au second seuil REF2.

Selon une variante de réalisation, l'élément gonflable 8 pourrait être subdivisé en au moins deux parties indépendantes gonflables, éventuellement sélectivement gérées par le système de pompe et d'échappement 30 et le circuit logique 31 ou par d'autres.

Selon une autre variante de réalisation, le matelas 1 pourrait comprendre d'autres dispositifs de support 6 placés à d'autres endroits adaptés, éventuellement sélectivement gérés par le système de pompe et d'échappement 30 et le circuit logique 31 ou par d'autres.

Selon une autre variante de réalisation, le dispositif de support 6 pourrait être intégré à un autre objet tel que l'assise ou le dossier d'un fauteuil.

Selon une autre variante de réalisation, le dispositif de support 6 et la feuille de substrat 20 munie des enroulements métalliques 19 pourraient former un coussin indépendant, associé à un système de pompe et d'échappement 30 et une unité électronique 31 pour constituer un ensemble fonctionnel indépendant.

Selon une autre variante de réalisation, l'unité électronique 31 pourraient être munie d'une alarme, sonore ou lumineuse, et programmée de façon à activer cette alarme par exemple lorsque l'enfoncement d'au moins certaines des cellules creuses 8 dépasse une valeur prédéterminée, c'est-à-dire lorsque la distance d'au moins certaines des pastilles métalliques 28 par rapport aux enroulements métalliques 19 passe au-dessous d'une valeur prédéterminée.

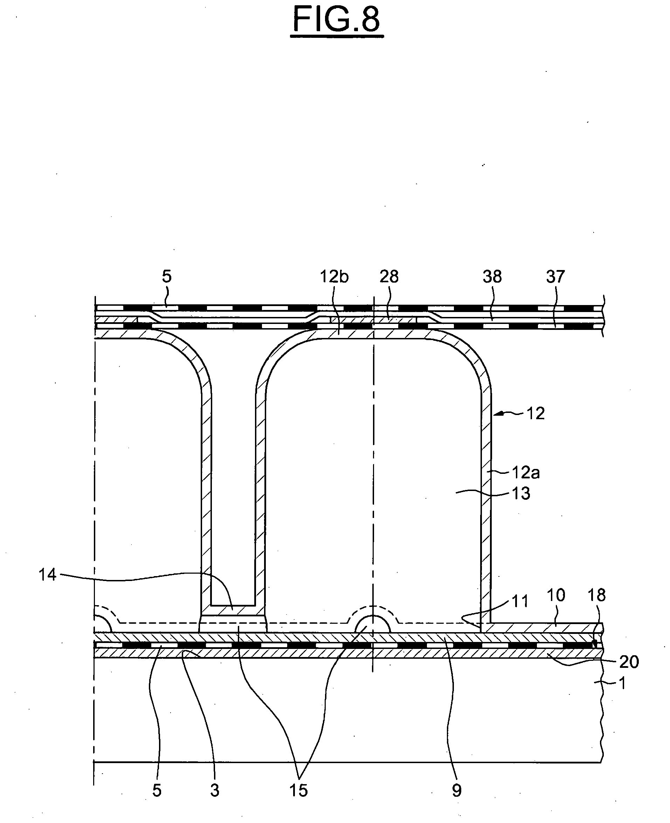

Selon une autre variante de réalisation illustrée sur la figure 8, les pastilles métalliques 28 ne sont plus portées par les extrémités des cellules gonflables 12 mais sont portées à plat par une feuille souple 37 s'étendant au-dessus des extrémités des cellules 12, les pastilles métalliques 28 étant disposées au-dessus des parties centrales plates des parois d'extrémité supérieure 12b des cellules 8. Par exemple, les pastilles métalliques 28 peuvent être insérées entre la feuille souple 37 et une autre feuille 38, ces feuilles étant reliées entre elles.

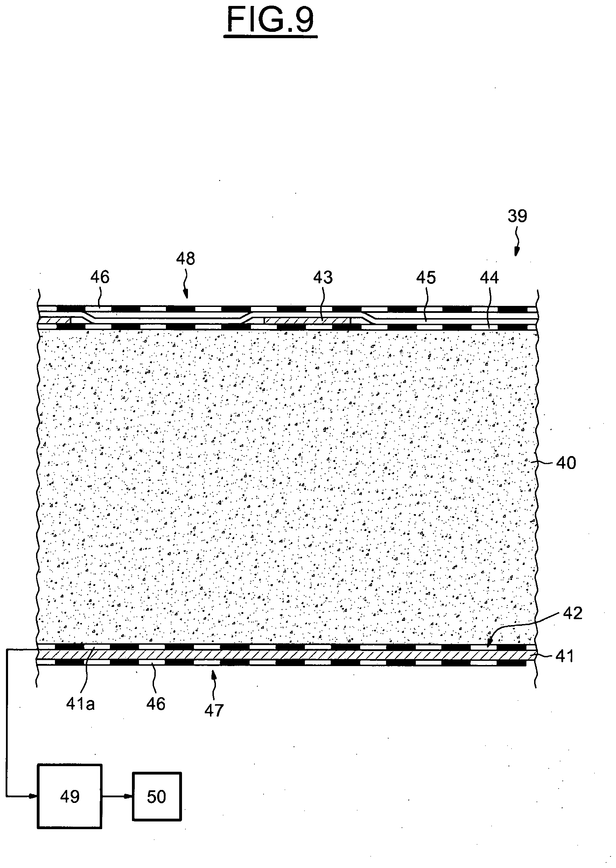

Comme illustré sur la figure 9, un autre dispositif de support 39 comprend un élément déformable formé par un bloc élastiquement déformable 40, par exemple en une mousse plastique expansée, présentant des faces opposées.

Le bloc élastiquement déformable 40 est équipé d'une feuille de substrat 41 associée à et placée au-dessus d'une face de ce bloc 40, avec interposition d'une feuille de protection 41a. Cette feuille de substrat 41 est équivalente à la feuille de substrat 20 de l'exemple précédent et est munie, comme cette feuille de substrat 20, d'une pluralité d'enroulements métalliques 42 à plat et répartis.

Le bloc élastiquement déformable 40 est également équipé d'une pluralité de pastilles métalliques réparties 43 associée à l'autre face de ce bloc 40 et disposées à plat, ces pastilles métalliques 43 étant équivalentes aux pastilles métalliques 28 de l'exemple précédent.

De façon équivalente à l'exemple de la figure 8, les pastilles métalliques 43 sont par exemple insérées entre deux feuilles souples 44 et 45 reliées entre elles.

L'ensemble ci-dessus peut être inséré dans une housse extérieure 46.

Le dispositif de support 39 ainsi constitué peut former un coussin ou un matelas ou une partie de ces derniers et présente une face d'appui 47 du côté de la feuille de substrat 41 pouvant être placée en appui sur un support extérieur et une face 48 du côté des pastilles métalliques 43 sur laquelle une personne peut venir en appui.

De façon équivalente à l'exemple précédent, les enroulements métalliques 42 portés par la feuille de substrat 41 sont reliés à une unité électronique 49 apte, de façon équivalente à l'unité électronique 31, à détecter l'enfoncement du bloc élastiquement déformable 40 par la détection du rapprochement d'au moins certaines des pastilles métalliques 43 vers les enroulements métalliques 42. L'unité électronique 49 peut être programmée pour activer une alarme 50, sonore ou lumineuse, dans le cas où ce rapprochement atteint un seuil.

Une telle disposition peut être utilisée dans le but d'indiquer si le dispositif de support 39 est mal adapté à la personne utilisatrice.

Selon une variante de réalisation, les enveloppes et les feuilles souples mentionnées plus haut peuvent être par exemple en un tissu élastique ou extensible.

Les dispositifs de support et les moyens de détection de déformations qui viennent d'être décrits présentent les avantages de réaliser des détections plus homogènes et reproductibles, d'être peu sensible ou insensible aux éventuelles perturbations électromagnétiques extérieures, d'être simple à réaliser et de pouvoir être protégé sans difficulté contre les agressions physiques extérieures.

La présente invention ne se limite pas aux exemples ci-dessus décrits. Bien d'autres variantes de réalisation sont possibles, sans sortir du cadre de l'invention.

## Revendications

1. Dispositif de support pour supporter un corps ou une partie d'un corps, en particulier d'un corps humain, comprenant au moins un élément déformable présentant une première face et une seconde face opposées et équipé d'au moins un moyen de détection de déformations dudit élément déformable,
dans lequel l'élément déformable (6) comprend une feuille d'embase (7) et au moins une pluralité de cellules creuses gonflables (8) en saillie d'un côté de la feuille d'embase et individuellement déformables en hauteur par rapport à la feuille d'embase,
dans lequel le moyen de détection comprend :
une feuille de substrat (20) associée à ladite feuille d'embase (7), s'étendant parallèlement à celle-ci, et munie d'une pluralité d'enroulements métalliques (19) formés à plat sur au moins une face de cette feuille de substrat,
et une pluralité de pastilles métalliques (28) solidaires respectivement des parties d'extrémité desdites cellules creuses gonflables (8), éloignées de ladite feuille d'embase (7),
et dans lequel les pastilles métalliques (28) sont respectivement situées au-dessus des parties centrales des enroulements métalliques (19).

2. Dispositif selon la revendication 1, dans lequel le moyen de détection de déformations comprend un moyen de détection inductive par impulsion relié auxdits enroulements.

3. Dispositif selon la revendication 2, dans lequel le moyen de détection de déformations comprend un moyen (34) permettant de relier séquentiellement et successivement les enroulements ou des groupes d'enroulements audit moyen de détection inductive par impulsion.

4. Dispositif selon l'une quelconque des revendications précédentes, dans lequel les enroulements (19) sont divisés en groupes d'enroulements (26, 27) dans lesquels les enroulements sont électriquement branchés en série..

5. Dispositif selon l'une quelconque des revendications 1 à 4, dans lequel les pastilles métalliques (28) sont portées par au moins une feuille souple (37) s'étendant au-dessus des parties d'extrémité desdites cellules creuses gonflables (8).

6. Dispositif selon l'une quelconque des revendications 1 à 4, dans lequel les pastilles métalliques (28) sont fixées sur les parties d'extrémité desdites cellules creuses gonflables (8).

7. Dispositif selon la revendication 6, dans lequel les pastilles métalliques (28) sont fixées sur une face intérieure des cellules creuses, parallèle à ladite feuille d'embase.

8. Dispositif selon l'une quelconque des revendications précédentes, dans lequel la feuille de substrat (20) est située sur le côté de la feuille d'embase (7) opposé auxdites cellules creuses.

9. Dispositif selon l'une quelconque des revendications précédentes, dans lequel les espaces intérieurs (13) des cellules creuses gonflables (8) sont reliés par l'intermédiaire de canaux (15) aménagés dans la feuille d'embase (7).

10. Dispositif selon l'une quelconque des revendications précédentes, dans lequel l'élément de gonflage comprend une première et une seconde feuilles (9, 10) accolées formant ladite feuille d'embase et une pluralité de protubérances creuses reliées à ladite seconde feuille (10) et délimitant lesdites cellules creuses, au moins l'une des feuilles étant conformée de façon à former des canaux de communication (15) entre les espaces intérieurs (13) des cellules creuses.

11. Dispositif selon l'une quelconque des revendications précédentes, dans lequel l'élément déformable comprend un bloc élastiquement déformable (40), les pastilles métalliques (43) étant portées par au moins une feuille souple (44).

12. Ensemble comprenant un dispositif de support selon l'une quelconque des revendications précédentes, et comprenant un système de pompe et d'échappement (30) relié à un moyen de branchement extérieur relié auxdites cellules creuses (8) par des canaux (15) inclus dans la feuille d'embase et une unité (31) électronique de détection inductive par impulsion reliée à des moyens de connexion électrique extérieure (22) reliés auxdits enroulements (19) et de gestion de l'activation du système de pompe et d'échappement (30).

13. Ensemble comprenant un dispositif de support selon l'une quelconque des revendications 1 à 11, et comprenant une unité (31) électronique de détection inductive par impulsion reliée à des moyens de connexion électrique extérieure (22) reliés auxdits enroulements (19) et de gestion d'une alarme.

## Patentansprüche

1. Trägervorrichtung, um einen Körper oder einen Körperteil, insbesondere eines menschlichen Körpers, zu unterstützen, umfassend mindestens ein verformbares Element, das eine erste Seite und eine zweite Seite, die einander gegenüberliegen, aufweist, und ausgestattet mit mindestens einem Mittel zur Erfassung von Verformungen des verformbaren Elements,
bei der das verformbare Element (6) eine Bodenplatte (7) und mindestens eine Vielzahl von aufblasbaren Hohlzellen (8) umfasst, die auf einer Seite der Bodenplatte herausragen und individuell in Bezug zur Bodenplatte in der Höhe verformbar sind,
wobei das Erfassungsmittel umfasst:
eine Substratplatte (20), die der Bodenplatte (7) zugeordnet ist, die sich parallel zu dieser erstreckt und mit einer Vielzahl von metallischen Wicklungen (19) versehen ist, die flach auf mindestens einer Seite der Substratplatte ausgebildet sind,
und eine Vielzahl von Metallplättchen (28), die jeweils mit den Endabschnitten der aufblasbaren Hohlzellen (8) verbunden sind, die von der Bodenplatte (7) entfernt sind,
und bei der die Metallplättchen (28) jeweils über den zentralen Abschnitten der metallischen Wicklungen (19) angeordnet sind.

2. Vorrichtung nach Anspruch 1, bei der das Mittel zur Erfassung von Verformungen ein Mittel zur induktiven Impulserfassung umfasst, das mit den Wicklungen verbunden ist.

3. Vorrichtung nach Anspruch 2, bei der das Mittel zur Erfassung von Verformungen ein Mittel (34) umfasst, das es ermöglicht, sequentiell und nacheinander die Wicklungen oder Wicklungsgruppen mit dem Mittel zur induktiven Impulserfassung zu verbinden.

4. Vorrichtung nach einem der vorhergehenden Ansprüche, bei der Wicklungen (19) Wicklungsgruppen (26, 27) unterteilt sind, in denen die Wicklungen in Serie elektrisch angeschlossen sind.

5. Vorrichtung nach einem der Ansprüche 1 bis 4, bei der die Metallplättchen (28) von mindestens einer flexiblen Platte (37) getragen werden, die sich über den Endabschnitten der aufblasbaren Hohlzellen (8) erstreckt.

6. Vorrichtung nach einem der Ansprüche 1 bis 4, bei der die Metallplättchen (28) an den Endabschnitten der aufblasbaren Hohlzellen (8) befestigt sind.

7. Vorrichtung nach Anspruch 6, bei der die Metallplättchen (28) an einer Innenseite der Hohlzellen parallel zur Bodenplatte befestigt sind.

8. Vorrichtung nach einem der vorhergehenden Ansprüche, bei der die Substratplatte (20) auf der Seite der Bodenplatte (7) gegenüber den Hohlzellen angeordnet ist.

9. Vorrichtung nach einem der vorhergehenden Ansprüche, bei der die Innenräume (13) der aufblasbaren Hohlzellen (8) mit Hilfe von Kanälen (15), die in der Bodenplatte (7) angeordnet sind, verbunden sind.

10. Vorrichtung nach einem der vorhergehenden Ansprüche, bei der das Aufblaselement eine erste und eine zweite aneinandergefügte Platte (9, 10) umfasst, die die Bodenplatte bilden, und eine Vielzahl von hohlen Ausstülpungen umfasst, die mit der zweiten Platte (10) verbunden sind und die Hohlzellen begrenzen, wobei mindestens eine der Platten ausgebildet ist, um Verbindungskanäle (15) zwischen den Innenräumen (13) der Hohlzellen zu bilden.

11. Vorrichtung nach einem der vorhergehenden Ansprüche, bei der das verformbare Element einen elastisch verformbaren Block (40) umfasst, wobei die Metallplättchen (43) von mindestens einer flexiblen Platte (44) getragen werden.

12. Einheit, umfassend eine Trägervorrichtung nach einem der vorhergehenden Ansprüche und umfassend ein Pump- und Ablasssystem (30), das mit einem externen Anschlussmittel verbunden ist, das mit den Hohlzellen (8) durch Kanäle (15) verbunden ist, die in die Bodenplatte eingeschlossen sind, und eine elektronische Einheit (31) zur induktiven Impulserfassung, die mit externen elektrischen Anschlussmitteln (22) verbunden ist, die mit den Wicklungen (19) verbunden sind, und zur Steuerung der Aktivierung des Pump- und Ablasssystems (30).

13. Einheit, umfassend eine Trägervorrichtung nach einem der Ansprüche 1 bis 11, und umfassend eine elektronische Einheit zur induktiven Impulserfassung (31), die mit externen elektrischen Anschlussmitteln (22) verbunden ist, die mit den Wicklungen (19) verbunden ist, und zur Erfassung eines Alarms.

## Claims

1. Support device for supporting a body or part of a body, in particular a human body comprising at least one deformable element that has a first face and an opposing second face, and that is equipped with at least one means for detecting deformations of the deformable element,
in which the deformable element (6) comprises a base sheet (7) and at least one plurality of hollow inflatable cells (8) that project from one side of the base sheet and are individually deformable height-wise with respect to the base sheet,
in which the detection means comprises:
a substrate sheet (20) associated with said base sheet (7) and extending parallel thereto and provided with a plurality of metal coils (19) formed flat on at least one face of this substrate sheet,
and a plurality of metal tabs (28) that are respectively secured to the end portions of said hollow inflatable cells (8) and remote from said base sheet (7),
and in which the metal tabs (28) are respectively located above the central portions of the metal coils (19).

2. Device according to Claim 1, in which the deformation detection means comprises a means for inductive detection by pulse connected to said coils.

3. Device according to Claim 2, in which the deformation detection means comprises a means (34) with which it is possible to connect, sequentially and successively, the coils or groups of coils to said means for inductive detection by pulse.

4. Device according to any one of the preceding claims, in which the coils (19) are divided into groups of coils (26, 27) in which the coils are connected electrically in series.

5. Device according to any one of Claims 1 to 4, in which the metal tabs (28) are borne by at least one flexible sheet (37) extending above the end portions of said hollow inflatable cells (8).

6. Device according to any one of Claims 1 to 4, in which the metal tabs (28) are secured to the end portions of said hollow inflatable cells (8).

7. Device according to Claim 6, in which the metal tabs (28) are secured to an internal face of the hollow cells, parallel to said base sheet.

8. Device according to any one of the preceding claims, in which the substrate sheet (20) is located on the opposite side of the base sheet (7) from said hollow cells.

9. Device according to any one of the preceding claims, in which the internal spaces (13) of the hollow inflatable cells (8) are connected by the intermediary of channels (15) created in the base sheet (7).

10. Device according to any one of the preceding claims, in which the inflation element comprises a first and a second sheet (9, 10) one on top of the other, forming said base sheet, and a plurality of hollow protuberances connected to said second sheet (10) and delimiting said hollow cells, at least one of the sheets being formed so as to create communication channels (15) between the internal spaces (13) of the hollow cells.

11. Device according to any one of the preceding claims, in which the deformable element comprises an elastically deformable block (40), the metal tabs (43) being borne by at least one flexible sheet (44).

12. Assembly comprising a support device according to any one of the preceding claims, and comprising a pump and vent system (30) connected to an external connection means connected to said hollow cells (8) by channels (15) included in the base sheet and an electronic unit (31) for inductive detection by pulse connected to means (22) for external electrical connection that are connected to said coils (19) and for managing activation of the pump and vent system (30).

13. Assembly comprising a support device according to any one of Claims 1 to 11, and comprising an electronic unit (31) for inductive detection by pulse connected to means (22) for external electrical connection that are connected to said coils (19) and for managing an alarm.
